(19) Europäisches Patentamt
European Patent Office
Office européen des brevets

(11) Veröffentlichungsnummer: **0 092 730**
**A2**

(12) **EUROPÄISCHE PATENTANMELDUNG**

(21) Anmeldenummer: **83103492.1**

(22) Anmeldetag: **11.04.83**

(51) Int. Cl.³: **C 07 D 249/08**
C 07 D 403/06, A 01 N 43/64

(30) Priorität: **24.04.82 DE 3215360**

(43) Veröffentlichungstag der Anmeldung:
**02.11.83 Patentblatt 83/44**

(84) Benannte Vertragsstaaten:
**AT BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Rentzea, Costin, Dr.**
**Neuenheimer Landstrasse 72**
**D-6900 Heidelberg(DE)**

(72) Erfinder: **Meyer, Norbert, Dr.**
**Stahlbuehlring 155A**
**D-6802 Ladenburg(DE)**

(72) Erfinder: **Jung, Johann, Dr.Dipl.-Landwirt**
**Hardenburgstrasse 19**
**D-6703 Limburgerhof(DE)**

(54) **Bisazolyl-Verbindungen, ihre Herstellung, ihre Verwendung zur Regulierung des Pflanzenwachstums und Mittel dafür.**

(57) Verbindungen der Formel I

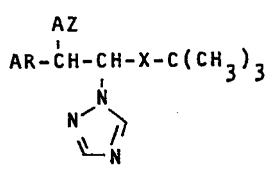

I

in der

Ar einen Naphthyl- oder Phenylrest bedeuten, wobei der Phenylrest durch Fluor-, Chlor-, Brom- oder Iod-Atome, Nitro, der Trifluormethylgruppen sowie durch Alkyl-, Alkoxy- oder Alkenylreste mit jeweils 1 bis 5 C-Atomen und ferner durch Phenoxyreste substituiert sein kann und

X eine $-CO-$, $-CH(OH)-$ oder $-CH(OR^3)$-Gruppe bedeutet, wobei $R^3$ einen Alkylrest mit 1 bis 8 C-Atomen oder einen gegebenenfalls durch Chlor substituierten Alkenylrest mit 2 bis 5 C-Atomen oder einen Alkinylrest mit 3 bis 4 C-Atomen oder Benzyl bedeutet, wobei der Benzylrest durch Fluor-, Chlor- oder Bromatome Nitro- oder Trifluormethylgruppen ferner durch Alkyl- oder Alkoxyreste mit 1 bis 4 C-Atomen substituiert sein kann, oder $R^3$ einen $-CO-R^4$ Rest bedeutet, wobei $R^4$ einen gegebenenfalls durch Halogenatome, Alkoxyreste, zweifach gebundenen Sauerstoff oder Carboxyalkylgruppe substituierten Alkylrest mit 1 bis 5 C-Atomen oder einen Phenylrest bedeutet, und

AZ einen 1-Imidazolyl, 1-Pyrazolyl- oder 1,2,4-Triazolylrest bedeutet sowie deren für Pflanzen verträgliche Salze und Metallkomplexe, deren Herstellung und Verwendung als wachstrumsregulierende Verbindungen.

EP 0 092 730 A2

Bisazolyl-Verbindungen, ihre Herstellung, ihre Verwendung zur Regulierung des Pflanzenwachstums und Mittel dafür

Die vorliegende Erfindung betrifft neue Bisazolyl-Verbindungen, Verfahren zu ihrer Herstellung, Mittel zur Regulierung des Pflanzenwachstums, die diese Verbindungen enthalten, und Verfahren zur Regulierung des Pflanzenwachstums mit diesen Verbindungen.

Es ist bekannt, daß bestimmte 2-Halogen-ethyl-trialkylammonium-halogenide pflanzenwachsregulierende Eigenschaften aufweisen (vgl. US-PS 3 156 554). So läßt sich mit Hilfe von (2-Chorethyl)-trimethylammoniumchlorid das Pflanzenwachstum beeinflussen. Allerdings ist die spezifische Wirksamkeit dieses Stoffes nicht immer ausreichend.

Es ist ferner bekannt, 3,3-Dimethyl-2-(1,2,4-triazolyl--(1))-1-(4-chlorbenzoyl)-butan zur Regulierung des Pflanzenwachstums zu verwenden (DE-OS 27 39 352).

Es wurde nun gefunden, daß Verbindungen der Formel I

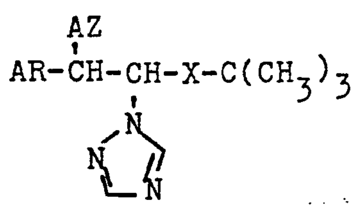

I

in der

Ar einen Naphthyl- oder Phenylrest bedeuten, wobei der Phenylrest durch Fluor-, Chlor-, Brom- oder Iod-Atome, Nitro, der Trifluormethylgruppen sowie durch Alkyl-,

Alkoxy- oder Alkenylreste mit jeweils 1 bis 5 C-Atomen und ferner durch Phenoxyreste substituiert sein kann und

X eine -CO-, -CH(OH)- oder $-CH(OR^3)$-Gruppe bedeutet, wobei $R^3$ einen Alkylrest mit 1 bis 8 C-Atomen oder einen gegebenenfalls durch Chlor substituierten Alkenylrest mit 2 bis 5 C-Atomen oder einen Alkinylrest mit 3 bis 4 C-Atomen oder Benzyl bedeutet, wobei der Benzylrest durch Fluor-, Chlor- oder Bromatome Nitro- oder Trifluormethylgruppen ferner durch Alkyl- oder Alkoxyreste mit 1 bis 4 C-Atomen substituiert sein kann, oder $R^3$ einen $-CO-R^4$ Rest bedeutet, wobei $R^4$ einen gegebenenfalls durch Halogenatome, Alkoxreste, zweifach gebundenen Sauerstoff oder Carboxyalkylgruppe substituierten Alkylrest mit 1 bis 5 C-Atomen oder einen Phenylrest bedeutet, und

AZ einen 1-Imidazolyl, 1-Pyrazolyl- oder 1,2,4-Triazolylrest bedeutet sowie deren für Pflanzen verträgliche Salze und Metallkomplexe hervorragend zur Beeinflussung des Pflanzenwachstums geeignet sind und eine sehr gute Pflanzenverträglichkeit besitzen.

Die neuen Verbindungen der Formel I enthalten mehrere chirale Zentren und werden im allgemeinen in Form von Racematen oder als Diastereomerengemische von erythro- sowie threo-Formen erhalten. Die erythro- und threo-Diasteremeren lassen sich bei einigen der erfindungsgemäßen Verbindungen beispielsweise durch Löslichkeitsunterschiede oder durch Säulenchromatographie trennen und in reiner Form isolieren. Aus solchen einheitlichen Diastereomerenpaaren kann man mit bekannten Methoden einheitliche Enantiomere erhalten. Diese werden von der vorliegenden Erfindung ebenfalls umfaßt. Als Mittel zur Beeinflussung des Pflanzenwachstums kann man sowohl die einheitlichen Diastereomeren bzw. Enantiomeren wie auch deren bei der

Synthese anfallenden Gemische verwenden. Aus wirtschaftlichen Gründen werden die letzteren bevorzugt verwendet, jedoch ist in einigen Fällen die spezifische Wirkung der reinen Raumformen höher als die ihrer Gemische, so daß die Reingewinnung sich lohnen kann.

Ar bedeutet beispielsweise 2-Furanyl, 2- und 3-Thienyl, 4-Biphenylyl, 1- und 2-Naphthyl, Phenyl, 4-Fluorphenyl, 2-Chlorphenyl, 3-Chlorphenyl, 4-Chlorphenyl, 4-Bromphenyl, 2,4-Dichlorphenyl, 3,4-Dichlorphenyl, 3,5-Dichlor-2-methoxyphenyl, 2,3,4-Trichlorphenyl, 2-Methoxyphenyl, 2,4-Dimethoxyphenyl, 3-Methoxyphenyl, 4-Methoxyphenyl, 4-Ethoxyphenyl, 4-tert.-Butoxyphenyl, 4-Methylphenyl, 4-Ethylphenyl, 4-Isopropylphenyl, 4-tert.-Butylphenyl, 3-Nitrophenyl, 4-Nitrophenyl, 3-Trifluormethylphenyl, 4-Trifluormethylphenyl und 4-Phenoxyphenyl.

X bedeutet beispielsweise eine Carbonylgruppe (C=O) oder die daraus durch Reduktion resultierenden Alkohole (-CHOH), die man weiterhin verethern (zu $-CH-OR^3$) oder verestern (zu $-CH-OCOR^4$) kann. Dabei bedeutet beispielsweise $R^3$ Methyl, Ethyl, n-Propyl, n-Butyl, 4-Chlrobutyl, n-Pentyl, n-Hexyl, Allyl, Buten-2-yl, Propargyl, Benzyl, 4-Fluorbenzyl, 4-Chlorbenzyl, 4-Brombenzyl, 2,4-Dichlorbenzyl und 4-Trifluormethylbenzyl. $R^4$ bedeutet vorzugsweise Methyl, Ethyl, n-Propyl, Isopropyl, Chlormethyl, Chlorpropyl, Methoxymethyl, Vinyl und Phenyl.

Die neuen Verbindungen lassen sich herstellen, indem man entweder

a) ein Triazolylketon der Formel (II)

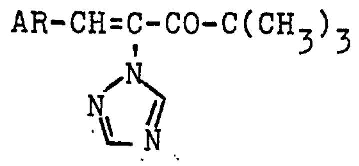

II

in der AR die oben angegebene Bedeutung hat, mit Imidazol oder Pyrazol oder 1,2,4-Triazol oder

b) ein Triazolylketon der Formel (III)

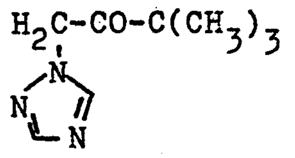

III

mit einem Aldehyd der Formel

AR-CHO IV

worin Ar die oben angegebene Bedeutung hat, mit Imidazol oder Pyrazol oder 1,2,4-Triazol, umsetzt und das so erhaltene Keton gegebenenfalls zum Alkohol reduziert und diesen gegebenenfalls verethert oder verestert. Die Reihenfolge der Umsetzungen kann u.U. auch vertauscht werden.

Die Umsetzung geschieht vorzugsweise in Gegenwart eines Lösungsmittels und gegebenenfalls unter Zusatz einer anorganischen oder organischen Base bei einer ausreichenden Temperatur im allgemeinen unter 120°C. Geeignete Lösungsmittel sind beispielsweise Alkohole wie Methanol, Ethanol, Propanol, Isopropanol, n-Butanol, Methylglykol oder Isoamylalkohol; Ether wie Diethyl-, Dipropyl-, Dibutylether, Tetrahydrofuran, Dioxan oder Dimethoxyethan; chlorierte Kohlenwasserstoffe wie Methylenchlorid, Dichlorethan, 1,1,1-Trichlorethan oder Chlorbenzol; Kohlenwasserstoffe wie Benzol, Toluol oder Xylol; Amide wie Dimethylformamid, Diethylformamid, Dimethylacetamid oder N-Methylpyrrolidon; Sulfoxide wie Dimethylsulfoxid und schließlich Sulfolan. Dabei ist es zweckmäßig, eine Base wie beispielsweise Natriumhydroxid, Kaliumhydroxid, Bariumhydroxid, Amine wie Triethylamin, N-Methylpiperidin oder N,-Dimethylcyclohexylamin zuzusetzen. Triazolylketone (II) sind bekannt (vgl. Jap. Patentanm. 53 130 661).

Die Umsetzung b) verläut vorzugsweise in Gegenwart eines des vorgenannten Lösungsmittels und unter Zusatz einer starken anorganischen oder organischen Base bei einer ausreichenden im allgemeinen unterhalb von 120°C.

Auch hierbei ist der Zusatz einer Base wie Natrium-, Kalium-, Calcium- und Bariumhydroxid, Natrium- und Kaliumcarbonat, Alkalialkoholate wie Natriumethylat, -ethylat, -isopropylat, Kalium-tert.-butoxid, Triethylamin, N-Methylpiperidin oder N,N-Dimethylcyclohexylamin empfehlenswert. Manchmal genügt es, schwache Basen wie Natrium- und Kaliumacetat zu verwenden.

1-(1,2,4-Triazolyl-(1)-3,3-dimethyl-butan-2-one (III) sind bekannt (DE-OS 26 38 479).

Die gemäß a) oder b) erhaltenen Ketone lassen sich gegebenenfalls in Gegenwart eines Lösungsmittels, bei Temperaturen zwischen -20 bis 150°C, bei Normaldruck oder unter erhöhtem Druck mit Wasserstoff in Gegenwart eines Katalysators, mit komplexen Bor- bzw. Aluminiumhydriden, mit Aluminiumisopropoxid und Isopropanol, mit Natriumdithionit oder elektrochemisch reduzieren.

Geeignete Lösungs- oder Verdünnungsmittel hierfür sind beispielsweise Wasser, Methanol, Ethanol, Isopropanol, Essigsäure, Diethylether, Tetrahydrofuran, Dioxan, Essigester, Toluol, Dimethylformamid oder deren Gemische.

Zur katalytischen Hydrierung verwendet man z.B. Platin- oder Palladiumkatalysatoren auf inerten Trägern und hydriert bei Drucken von bis zu 80 bar bis keine Wasserstoffaufnahme mehr erfolgt.

Für die als Reduktionsmittel verwendbaren Hydriede seien beispielsweise Natriumborhydrid oder Lithiumaluminiumhydrid genannt.

Die erhaltenen Alkohole (I) (X = CHOH) lassen sich mit Alkylierungsmitteln V

$$Z\text{-}R^3, \qquad V$$

worin $R^3$ die oben angegebene Bedeutung hat und Z Chlor oder Brom darstellt, gegebenenfalls, in Gegenwart eines Lösungsmittels in ein- oder zweiphasiger Umsetzung, gegebenenfalls unter Zusatz einer anorganischen Base und gegebenenfalls unter Zusatz eines Reaktionsbeschleunigers und/oder eines Phasentransferkatalysators verethern. Es eignen sich die üblichen, oben erwähnten Lösungsmittel und Basen.

Geeignete Reaktionsbeschleuniger sind beispielsweise Metallhalogenide wie Natriumbromid, Natriumiodid, Kaliumbromid, Kaliumiodid, tertiäre Amine wie 4-Dimethylaminopyridin und 4-Pyrrolidinopyridin, Kronenether wie 12-Krone-4, 14-Krone-5, 18-Krone-6, Dibenzo-18-Krone-6 oder Dicyclohexano-18-Krone-6 oder Azole wie Imidazol und 1,2,4-Triazol.

Als Phasentransferkatalysatoren kommen vorzugsweise quaternäre Ammoniumsalze wie Tetrabutylammoniumchlorid, -bromid, -iodid oder -hydrogensulfat, Benzyl-ttiethylammoniumchlorid, Methyl-trioctylammoniumchlorid und -bromid und Phosphoniumsalze wie Tetrabutylphosphoniumbromid und -iodid und Tetra-n-pentylphosphoniumbromid und -iodid in Frage.

Die Alkohole (I) (X = CHOH) können mit Säurechloriden oder -anhydriden der Formeln $R^4$-COCl oder $(R^4\text{-CO})_2O$ gegebenen-

falls in Gegenwart eines säurebindenden Mittels und/oder eines Reaktionsbeschleunigers verestert werden. Hierfür eignen sich dieselben Lösungsmittel und Basen wie für die Veretherung. Zusätzlich können tertiäre Amine wie Trimethylamin, Triethylamin, N,N-Dimethylanilin, N,N-Dimethylcyclohexylamin, N-Methylpiperidin oder Pyridin als Base verwendet werden.

Mit geeigneten Basen können die Alkohole auch in einer ersten Umsetzung zunächst in ihre Alkoholat-Salze überführt werden und dann als solche zur Reaktion gebracht werden.

Die so erhaltenen Verbindungen (I) werden nach üblichen Methoden isoliert, gegebenenfalls gereinigt und gegebenenfalls mit Säuren oder Metallsalzen zu Salzen bzw. zu Metall-Komplexen umgesetzt.

Beispiel 1

a) 51 g (0,2 Mol) 1-Phenyl-2-(1,2,4-triazolyl-(1)-4,4-dimethyl-1-penten-3-on, 41 g (0,6 Mol) 1,2,4-Triazol und 1,5 g Kaliumhydroxid werden in 150 ml Dimethylformamid gelöst und 2 Tage bei 80°C gerührt. Danach wird das Lösungsmittel i.V. abdestilliert, der Rückstand in 400 ml Methylenchlorid gelöst, dreimal mit je 100 ml Wasser gewaschen, über $Na_2SO_4$ getrocknet und eingeengt. Der Rückstand wird in 50 ml Ether aufgenommen und 12 Stunden bei +5°C aufbewahrt. Der gebildete kristalline, farblose Niederschlag wird abgesaugt, mit 100 ml n-Pentan gewaschen und getrocknet. Es wurden 35 g (54 % d.Th.) 1,2-Bis(1,2,4-trazolyl-(1))1-phenyl-4,4-dimethylpentan-3-on vom Schmelzpunkt 208 bis 210°C erhalten.

b) 21,2 g (0,2 Mol) Benzaldehyd, 41 g (0,6 Mol) 1,2,4--Triazol, 33,4 g (0,2 Mol) 1-(1,2,4-Triazolyl-(1))--3,3-dimethylbutan-2-on und 5 g Piperidin werden in 250 ml Ethanol gelöst und 2 Tage bie 70°C gerührt. Nach Abkühlen auf 20°C wird mit 8 g Essigsäure versetzt und im Wasserstrahlvakuum eingeengt. Der Rückstand wird in 400 ml Methylenchlorid gelöst, viermal mit je 100 ml Wasser gewaschen, über $Na_2SO_4$ getrocknet und eingeengt. Der Rückstand wird mit 50 ml Ether bei +5°C digeriert, abgesaugt, mit wenig Ether gewaschen und getrocknet.

Es werden 33,1 g (51 % d.Th.) 1,2-Bis-(1,2,4-triazolyl-(1))-1-phenyl-4,4-dimethylpentan-3-on vom Schmelzpunkt 208 - 209°C erhalten.

Beispiel 2

In eine Lösung von 22,8 g des nach der vorstehenden Vorschrift erhaltenen (0,07 Mol) 1,2-Bis-(1,2,4-triazolyl--(1))-1-phenyl-4,4-dimethylpentan-3-on in 250 ml Methanol werden unter Rühren und Eiskühlung bei 3 bis 8°C protionsweise 3,8 g (0,1 Mol) Natriumborhydrid eingetragen. Nach 12stündigem Rühren bei Raumtemperatur wird eingeengt, der Rückstand 1 Stunde mit 400 ml Methylenchlorid und 70 ml 15%iger Kalilauge gerührt. Die organische Schicht wird dreimal mit je 100 ml Wasser gewaschen, über $Na_2SO_4$ getrocknet und eingeengt. Der verbleibende feste, farblose Rückstand wird mit 20 ml kaltem (-30°C) Ether und mit 50 ml n-Pentan gewaschen und getrocknet.

Es wurden 17 g (74,5 % d.Th.) 1,2-Bis-(1,2,4-triazolyl--(1))-1-phenyl-4,4-dimethylpentan-3-ol vom Schmelzpunkt 198 bis 208°C erhalten.

Beispiel 3

Eine Mischung aus 12 g der nach der vorstehenden Vorschrift erhaltenen (0,0368 Mol) 1,2-Bis-(1,2,4-triazolyl--(1)-1-phenyl-4,4-dimethylpentan-3-ol, 1 g 4-Dimethylaminopyridin und 60 g Propionsäureanhydrid werden 16 Stunden bei 60°C gerührt und anschließend in 250 ml Eiswasser eingerührt. Die wäßrige Suspension wird dreimal mit je 100 ml Methylenchlorid ausgeschüttelt. Die vereinigten Extrakte werden 30 Minuten mit 200 ml einer 6%igen Natriumhydrogencarbonatlösung gerührt, die organische Phase abgetrennt, über Natriumsulfat getrocknet und eingeengt. Der halbfeste, farblose Rückstand wird mit 50 ml Petrolether bei 20°C 12 Stunden digeriert und abgesaugt. Es weruden 6 g (42,7 % d.Th) 1,2-Bis-(1,2,4-triazolyl-(1))-1--phenyl-3-propionyloxy-4,4-dimethylpentan vom Schmelzpunkt 40 - 45°C erhalten.

Durch entsprechende Abwandlung der Ausgangsstoffe und Zwischenprodukte konnten die in der folgenden Tabelle 1 aufgeführten Verbindungen erhalten werden. Die Verbindungen, von denen kein Schmelzpunkt angegeben ist, wurden nicht zur Bestimmung des Schmelzpunkts isoliert.

0092730

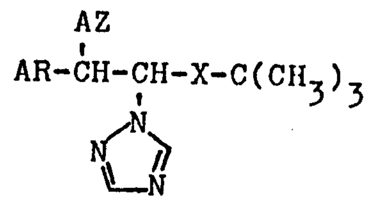

| Nr. | AR | AZ | X | $F_p$ [°C] |
|---|---|---|---|---|
| 4 | $C_6H_5$- | 1,2,4-Triazolyl-(1)- | $-CH-O-CO-C_3H_7$ | Harz |
| 5 | $C_6H_5$- | -"- | $-CH-O-CO-CH=CH_2$ | " |
| 6 | $C_6H_5$- | -"- | $-CH-O-CO-C_6H_5$ | " |
| 7 | $1-C_{10}H_7$- | -"- | -CO- | 172-178 |
| 8 | $1-C_{10}H_7$- | -"- | -CH-OH- | |
| 9 | $4-ClC_6H_4$- | -"- | -CO- | 157-159 |
| 10 | -"- (Diastereomer I) | -"- | -CH-OH | 247-250 |
| 11 | $4-ClC_6H_4$- (Diastereomerengemisch) | -"- | -"- | 91-95 |
| 12 | $4-ClC_6H_4$ | 1-Pyrazolyl | -CO- | 126-128 |
| 13 | -"- | -"- | -CH-OH | 144-145 |
| 14 | -"- | -"- | $-CH-O-CO-CH_3$ | Harz |
| 15 | -"- | 1-Imidazolyl | -CO- | 114-117 |
| 16 | -"- | -"- | -CH-OH | |
| 17 | $4-FC_6H_4$- | 1,2,4-Triazolyl-(1) | -CO- | 219-221 |

Tabelle (Forts.)

| Nr. | AR | AZ | X | $F_p$[°C] |
|---|---|---|---|---|
| 18 | 4-$FC_6H_4$- | 1,2,4-Trazolyl-(1)- | -CH-OH | 246-248 |
| 19 | -"- | -"- | -CH-O-CO-$C_6H_5$ | |
| 20 | 4-$BrC_6H_4$- | -"- | -CO- | |
| 21 | -"- | -"- | -CH-OH | |
| 22 | 4-$(CH_3)C_6H_4$- | -"- | -CO- | 165-167 |
| 23 | -"- | -"- | -CH-OH | 180-215 |
| 24 | 4-(tert.-$C_4H_9$)$C_6H_4$- | -"- | -CO- | 190-192 |
| 25 | -"- | -"- | -CH-OH | 233-238 |
| 26 | -"- | -"- | -CH-O-CO-$CH_2Cl$ | |
| 27 | 4-$(CH_3O)C_6H_4$- | -"- | -CO- | 180-182 |
| 28 | -"- | -"- | -CH-OH | Harz |
| 29 | -"- | -"- | -CH-O-CO-$CH_2$-O-$CH_3$ | |
| 30 | 4-$(CF_3)C_6H_4$- | -"- | -CO- | |
| 31 | -"- | -"- | -CH-OH | |
| 32 | -"- | -"- | -CH-O-CO-$C_2H_5$ | |
| 33 | 4-$(NO_2)C_6H_4$- | -"- | -CO- | |
| 34 | -"- | -"- | -CH-OH | |
| 35 | 3,4-$Cl_2C_6H_3$- | -"- | -CO- | 176-178 |
| 36 | -"- | -"- | -CH-OH | 158-168 |

Tabelle (Forts.)

| Nr. | AR | AZ | X | $F_p$[°C] |
|---|---|---|---|---|
| 37 | 2,4-$Cl_2C_6H_3$- | 1-Pyrazolyl | -CO- | 128-130 |
| 38 | -"- | -"- | -CH-OH | Harz |
| 39 | -"- | 1,2,4-Triazolyl-(1) | -CO- | 143-146 |
| | (Diastereomer I) | | | |
| 40 | 2,4-$Cl_2C_6H_3$- | -"- | -CH-OH | 212-214 |
| 41 | -"- | -"- | -CO- | 156-159 |
| | (Diastereomer II) | | | |
| 42 | 2,4-$Cl_2C_6H_3$- | -"- | -CH-OH | 170-190 |
| 43 | -"- | -"- | -CO- | 120-147 |
| | (Diastereomerengemisch) | | | |
| 44 | 2,4-$Cl_2C_6H_3$- | -"- | -CHOH | 169-172 |
| 45 | 4-(CN)$C_6H_4$- | -"- | -CO- | |

Die neuen Verbindungen können praktisch alle Entwicklungsstadien einer Pflanze beeinflussen und werden als Wachstumsregulatoren eingesetzt.

Die Wirkungsvielfalt der Pflanzenwachstumsregulatoren hängt ab vor allem

a) von der Pflanzenart und -sorte,
b) von dem Zeitpunkt der Applikation, bezogen auf das Entwicklungsstadium der Pflanze und von der Jahreszeit,
c) von dem Applikationsort und -verfahren (Samenbeize, Bodenbehandlung oder Blattapplikation),
d) von den geoklimatischen Faktoren, z.B. Sonnenschein, Dauer, Durchschnittstemperatur, Niederschlagsmenge,
e) von der Bodenbeschaffenheit (einschließlich Düngung),
f) von der Formulierung bzw. Anwendungsform des Wirkstoffs und schließlich
g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern,

Kanalböschungen und auf Rasenflächen wie Park, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumshemmern läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch

die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkranheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den neuen Wirkstoffen lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die neuen Stoffe Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Wachstumsstadien als auch eine Beschleunigung bzw.

Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt und Sproßteil der Pflanze ist auch für ein gut kontrollierbares Entblättern der Bäume wesentlich.

Die Wirkung der neuen Verbindungen ist besser als bei bekannten Wachstumsregulatoren. Die Wirkung zeigt sich sowohl bei Monokotylen, z.B. Getreide wie Weizen, Gerste, Roggen, Hafer und Reis oder Mais oder Gräsern als auch insbesondere bei Dikotylen (z.B. Sonnenblumen, Tomaten, Erdnüssen, Reben, Baumwolle, Raps und vor allem Soja) und verschiedenen Zierpflanzen wie Chrysanthemen, Poinsettien und Hibiskus.

Die erfindungsgemäßen Wirkstoffe können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

ür die Blatt- und Bodenbehandlung sind im allgemeinen Gaben von 0,1 bis 12 kg/ha bevorzugt 0,25 bis 3 kg/ha als ausreichend zu betrachten.

Die erfindungsgemäßen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt. z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel zugesetzt werden können. Als Hilfsstoffe zur Formulierung kommen im wesentlichen in Frage Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin), Dimethylformamid und Wasser; feste Trägerstoffe wie natrüliche Gesteinsmehle (z.B. Kaoline, Tonerden, Talkum, Kreide) und synthetische Gesteinsmehle (z.B. hochdisperse Kieselsäure, Silikate); Emulgiermittel oder sonstige oberflächenaktive Mittel, wie nichtionogene und anionische Emulgatoren (z.B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemäßen Verbindungen in wäßriger Lösung gegebenenfalls unter Zusatz von mit Wasser mischbaren organischen Lösungsmitteln wie Methanol oder anderen niederen Alkoholen, Aceton, Dimethylformamid oder N-Methylpyrrolidin. Die Formulierungen

enthalten im allgemeinen zwischen 0,1 und 95 Gewichtsprozent Wirkstoff, vorzugsweise zwischen 0,5 und 90.

Die Formulierungen bzw. die daraus hergestellten gebrauchsfertigen Zubereitungen wie Lösungen, Emulsionen Suspensionen, Pulver, Stäube, Pasten oder Granulate werden in bekannter Weise angewendet, beispielsweise im vorauflaufverfahren, im Nachauflaufverfahren oder als Beizmittel.

Beispiele für Formulierungen sind:

I 20 Gewichtsteile der Verbindung des Beispiels 1 werden in 3 Gewichtsteilen des Natriumsalzes der Diisobutylnaphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

II 3 Gewichtsteile der Verbindung des Beispiels 2 werden mit 97 Gewichtsteilen feinteiligem Kaolin innig vermischt. Man erhält auf diese Weise ein Stäubemittel, das 3 Gew.% des Wirkstoffs enthält.

III 30 Gewichtsteile der Verbindung des Beispiels 10 werden mit einer Mischung aus 92 Gewichtsteile pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV 40 Gewichtsteile der Verbindung des Beispiels 33 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff enthält.

V 20 Teile der Verbindung des Beispiels 28 werden mit 2 Teilen Calciumsalz der Dodecylbenzolsulfonsäure, 8 Teile Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

VI Man vermischt 90 Gewichtsteile der Verbindung des Beispiels 1 mit 10 Gewichtsteilen N-Methyl-pyrrolidon und erhält eine Lösung, die zur Anwendung in Form kleinster Tropfen geeignet ist.

VII 20 Gewichtsteile der Verbindung des Beispiels 36 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

VIII 20 Gewichtsteile der Verbindung des Beispiels 13 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IX 20 Gewichtsteile der Verbindung des Beispiels 40 werden in einer Mischung gelöst, die aus 25 Gewichtsteilen Cyclohexanol, 65 Gewichtsteilen einer Mineralölfraktion vom Siedepuntk 210 bis 280°C und 10 Gewichtsteilen des Anlagerungsproduktes von 40 mol Ethylenoxid an 1 Mol Ricinusöl besteht. Durch Eingießen und feines Verteilen der Lösung in 100.000 Gewichtsteilen Wasser erhält man eine wäßrige Dispersion, die 0,02 Gew.-% des Wirkstoffs enthält.

Die erfindungsgemäßen Mittel können in diesen Aufwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Wachstumsregulatoren erhält man dabei in vielen Fällen eine Vergrößerung des Wirkungsspektrums. Bei einer Anzahl solcher Wachstumsregulatormischungen treten auch synergistische Effekte auf, d.h. die Wirksamkeit des Kombinationsproduktes ist größer als die addierten Wirksamkeiten der Einzelkomponenten.

Fungizide, die mit den erfindungsgemäßen Verbindungen kombiniert werden können, sind beispielsweise Dithiocarbamate und deren Derivate, wie
Ferritdimethyldithiocarbamat,
Zinkdimethyldithiocarbamat,
Manganethylenbisthiocarbamat,
Mangan-Zink-ethylendiamin-bis-dithiocarbamat,
Zinkethylenbisthiocarbamat,
Tetramethylthiuramdisulfide,
Ammoniak-Komplex von Zink-(N,N-ethylen-bis-dithiocarbamat) und
N,N'-Polyethylen-bis-(thiocarbamoyl)-disulfid,
Zink-(N,N'-propylen-bis-dithiocarbamat),
Ammoniak-Komplex von Zinn-(N,N'-propylen-bis-dithiocarbamat) und
N,N'-Polypropylen-bis-(thiocarbamoyl)-disulfid;

Nitrophenolderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Strukturen, wie
N-Trichlormethylthio-tetrahydrophthalimid,
N-Trichlormethylthio-phthalimid,
2-Heptadecyl-2-imidazol-acetat,
2,4-Dichlor-6-(o-chloranilino)-s-triazin,
O,O-Diethyl-phthalimidophosphonthionat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithiaanthrachinon,
2-Thio-1,3-dithio-(4,5-b)-chinoxalin,
1-Butylcarbamoyl-2-benzimidazol-carbaminsäuremethylester,
2-Methoxycarbonylamino-benzimidazol,

2-Rhodanmethylthio-benzthiazol,
4-(2-Chlorphenylhydrazonon)-3-methyl-5-isoxazolon,
Pyridin-2-thiol-1-oxid,
8-Hydrochinolin bzw. dessen Kupfersalz,
2,3-Dihydro-5-carboxynilido-6-methyl-1,4-oxathiin-4,4-dioxid,
2,3-Dihydro-5-carboxanilido-6-methyl-1,4-oxathiin,
2-(Furyl-2)-benzimidazol,
Piperazin-1,4-diyl-bis-1-(2,2,2-trichlor-ethyl)-formamid,
2-Thiazolyl-(4)-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

und verschiedene Fungizide, wie
Dodecylguanidinacetat,
3-(2-(3,5-Dimethyl-2-oxycyclohexyl)-2-hydroxyethyl)-glutarimid,
Hexachlorbenzol,
N-Dichlorfluormethylthio-N,N'-dimethyl-N-phenyl-schwefelsäurediamid,
D,L-Methyl-N-(2,6-dimethyl-phenyl)-N-furyl(2)-alaninat,
D,L-N-(2,6-Dimethyl-phenyl)-N-(2'-methoxyacetyl)-alaninmethylester,
5-Nitro-isophthalsäure-di-isopropylester,
2,5-Dimethyl-furan-3-carbonsäureanilid,
2,5-Dimethyl-furan-3-carbonsäure-cyclohexylamid,
2-Methyl-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
2,3-Dichlor-1,4-naphthochinon,
1,4-Dichlor-2,5-dimethoxybenzol,

p-Dimethylaminobenzol-diazinnatriumsulfonat,
1-Chlor-2-nitro-propan,

Polychlornitrobenzole, wie Pentachlornitrobenzol, Methylisocyanat, fungizide Antibiotika, wie Griseofulvin oder Kasugamycin, Tetrafluordichloraceton, 1-Phenylthiosemicarbazid, Bordeauxmischung, nickelhaltige Verbindungen und Schwefel.

In den nachfolgenden Anwendungsbeispielen wird die Wirkung erfindungsgemäßer Verbindungen als Pflanzenwachstumsregulatoren dargestellt, ohne die Möglichkeit weiterer Anwendungen auszuschließen.

Anwendungsbeispiele

Reis, Sorte "Girona"; Nachauflaufverfahren

| Stoffbeispiele | Aufwandmengen mg WS/Gefäß | Wuchshöhen rel. |
|---|---|---|
| unbehandelt | - | 100 |
| Chlorcholinchlorid (CCC; Vergleich) | 1,5 | 98,9 |
| | 6,0 | 94,6 |
| 10 | 1,5 | 83,1 |
| | 6,0 | 78,8 |

Sommerraps, Sorte "Petranova"; Vorauflaufverfahren

| Stoff-beispiele | Aufwandmengen mg WS/Gefäß | Wuchshöhen rel. |
|---|---|---|
| unbehandelt | - | 100 |
| CCA | 3 | 96,0 |
| | 12 | 96,0 |
| 10 | 3 | 96,0 |
| | 12 | 88,4 |
| 11 | 3 | 80,8 |
| | 12 | 55,6 |
| 12 | 3 | 90,2 |
| | 12 | 61,0 |

Sommerraps, Sorte "Petranova"; Nachauflaufverfahren

| Stoff-beispiele | Aufwandmengen mg WS/Gefäß | Wuchshöhen rel. |
|---|---|---|
| unbehandelt | - | 100 |
| CCC | 1,5 | 96,6 |
| | 6,0 | 96,6 |
| 10 | 1,5 | 82,6 |
| | 6,0 | 78,0 |
| 17 | 1,5 | 89,8 |
| | 6,0 | 83,4 |
| 35 | 1,5 | 91,8 |
| | 6,0 | 82,1 |
| 40 | 1,5 | 91,8 |
| | 6,0 | 74,9 |

Soja, Sorte "Gieso"; Nachauflaufverfahren

| Stoffbeispiele | Aufwandmengen mg WS/Gefäß | Wuchshöhen rel. |
|---|---|---|
| unbehandelt | - | 100 |
| CCC | 0,5 | 94,7 |
| | 1,5 | 92,8 |
| 9 | 0,5 | 93,9 |
| | 1,5 | 84,5 |
| 10 | 0,5 | 70,4 |
| | 1,5 | 59,9 |

Patentansprüche

1. Verbindungen der Formel I

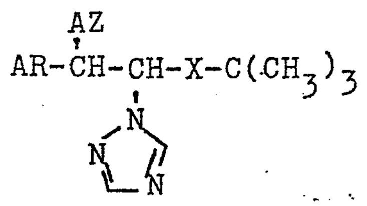

I

in der

Ar einen Naphthyl- oder Phenylrest bedeutet, wobei der Phenylrest durch Fluor-, Chlor-, Brom- oder Iod-Atome, Nitro, der Trifluormethylgruppen, sowie durch Alkyl-, Alkoxy- oder Alkenylreste mit jeweils 1 bis 5 C-Atomen und ferner durch Phenoxyreste substituiert sein kann und

X eine -CO-, -CH(OH)- oder $-CH(OR^3)$-Gruppe bedeutet, wobei $R^3$ einen Alkylrest mit 1 bis 8 C-Atomen oder einen gegebenenfalls durch Chlor substituierten Alkenylrest mit 2 bis 5 C-Atomen oder einen Alkinylrest mit 3 bis 4 C-Atomen oder Benzyl bedeutet, wobei der Benzylrest durch Fluor-, Chlor- oder Bromatome, Nitro- oder Trifluormethylgruppen ferner durch Alkyl- oder Alkoxyrest mit 1 bis 4 C-Atomen substituiert sein kann, oder $R^3$ einen $-CO-R^4$ Rest bedeutet, wobei $R^4$ einen gegebenenfalls durch Halogenatome, Alkoxyreste, zweifach gebundenen Sauerstoff oder eine Carboxyalkylgruppe substituierten Alkylrest mit 1 bis 5 C-Atomen oder einen Phenylrest bedeutet, und

AZ einen 1-Imidazolyl, 1-Pyrazolyl oder 1,2,4-Triazolylrest bedeutet, sowie deren für Pflanzen verträgliche Salze und Metallkomplexe.

2. Verbindungen der Formel I gemäß Anspruch 1, dadurch gekennzeichnet, daß

Ar 1- und 2-Naphthyl, Phenyl, 2- und 4-Fluorphenyl, 3- und 4-Chlorphenyl, 4-Bromphenyl, 4-Methoxyphenyl, 4-Ethoxyphenyl, 4-Propoxyphenyl, 4-Methylphenyl, 2,4-Dimethylphenyl, 4-Ethylphenyl, 4-Propylphenyl, 4-n-Butylphenyl, 4-Isobutylphenyl, 4-tert.-Butylphenyl, 4-Pentyl, 4-Phenoxyphenyl, 4-Nitrophenyl, 3- und 4-Trifluormethylphenyl, 4-Cyanphenyl, 4-Allylphenyl, 3,4-Dichlorphenyl, 2,4-Dichlorphenyl und 2,6-Dichlorphenyl,

X eine -CO-, -CH-(OH)- oder $-CH(OR^3)$-Gruppe bedeutet, wobei

$R^3$ Methyl, Ethyl, n-Propyl, n-Butyl, 4-Chlorbutyl, Allyl, 2-Buten-1-yl, Propargyl, Benzyl, 4-Fluorbenzyl, 4-Chlorbenzyl, 4-Brombenzyl, 4-Trifluormethylbenzyl, 4-Nitrobenzyl, 4-Methylbenzyl, 4-tert.-Butylbenzyl, 2,4-Dichlorbenzyl, 3,4-Dichlorbenzyl, 2,6-Dichlorbenzyl oder einen $-CO-R^4$ rest,

$R^4$ Methyl, Chlormethyl, Methoxymethyl, Ethyl, n-Propyl oder Phenyl,

AZ 1-Imidazolyl, 1-Pyrazolyl oder 1,2,4-Triazolyl-(1)- bedeuten.

3. Verfahren zur Hetstellung von Verbindungen der Formel I gemäß Anspruch 1, <u>dadurch gekennzeichnet</u>, daß man

a) ein Keton der Formel

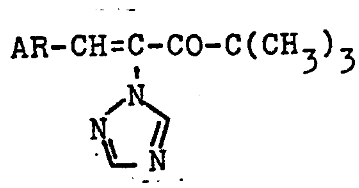

II

in der AR die in Anspruch 1 genannte Bedeutung besitzt, mit Imidazol oder Pyrazol oder 1,2,4-Triazol oder

b) ein Keton der Formel

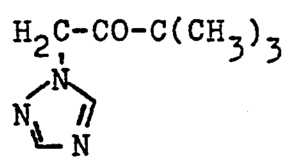

III

mit einem Aldehyd der Formel

AR-CHO IV

worin AR die in Anspruch 1 angegebene Bedeutung hat, mit Imidazol oder Pyrazol oder 1,2,4-Triazol gegebenenfalls in Gegenwart eines basischen Katalysators und gegebenenfalls in Gegenwart eines Lösungs- oder Verdünnungsmittels umsetzt, und das erhaltene Keton gegebenenfalls zum Alkohol reduziert und diesen gegebenenfalls verethert oder verestert.

4. Mittel zur Regulierung des Pflanzenwachstums, enthaltend eine oder mehrere Verbindungen gemäß Anspruch 1.

5. Mittel zur Regulierung des Pflanzenwachstums, enthaltend eine oder mehrere Verbindungen gemäß Anspruch 1 und einen flüssigen oder festen Trägerstoff sowie gegebenenfalls eine oder mehrere oberflächenaktive Mittel.

6. Verwendung von Verbindungen gemäß Anspruch 1 zur Regulierung des Pflanzenwachstums.

7. Verfahren zur Regulierung des Pflanzenwachstums, dadurch gekennzeichnet, daß man eine oder mehrere Verbindungen gemäß Anspruch 1 auf Pflanzen oder deren Lebensraum einwirken läßt.

8. Verfahren zur Herstellung von das Pflanzenwachstum regulierenden Mitteln, dadurch gekennzeichnet, daß man eine oder mehrere Verbindungen gemäß Anspruch 1 mit festen oder flüssigen Trägerstoffen sowie gegebenenfalls einem oder mehreren oberflächenaktiven Mitteln mischt.